# EUROPEAN PATENT APPLICATION

(11) **EP 1 053 760 A2**
(43) Date of publication of application: **22.11.2000**
(21) Application number: 00110529.5
(22) Date of filing: 17.05.2000
(51) Int. Cl.: A61M 1/36

(54) **Fully constrained soft shell reservoir**

(30) Priority: 21.05.1999 US 316874
(71) Applicant: Medtronic Inc., Minneapolis, MN 55432-3576 (US)
(72) Inventor: Elgas, Roger J., Ahaheim Hills, California 92802 (US); Petersen, Michael P., Eden Prairie, Minnesota 55437 (US)
(74) Representative: Hughes, Andrea Michelle

(57) **Abstract**

A fully constrained soft shell reservoir 12 uses a hard shell reservoir lined with a flexible liner 16 sealed to the hard shell reservoir along its top. Both the blood inlet 22 and the blood outlet 24 are placed in the bottom of the hard shell reservoir underneath the liner. The liner acts as a soft shell reservoir inside the hard shell reservoir, and thereby combines the advantages of both types of reservoirs. By enclosing the hard shell reservoir and applying a vacuum above the liner, assisted venous return can be accomplished. The inventive construction lends itself readily to integration of a cardiotomy reservoir with the venous reservoir.

## Description

This invention relates to venous reservoirs for use in cardiac surgery, and more particularly to a reservoir which combines the advantages of both a soft shell and a hard shell reservoir.

Venous reservoirs are used during heart surgery to hold blood from the patient's venous return line. Blood is pumped from the reservoir into a membrane oxygenator, from which it then flows back to the patient. Traditionally, two generic types of reservoirs have been used. The first type is the "soft shell", which uses a bag generally made from flexible sheets of plastic. Soft shell reservoirs have two advantages: 1) there is no exposure of venous return blood to air, and 2) the bags collapse when emptied, thereby minimizing the potential for pumping air into the patient if venous blood flow is interrupted for any reason. The disadvantages of soft shell reservoirs are that they do not provide for return of cardiotomy blood, and that they make accurate measurement of the blood volume they contain very difficult. The second type of reservoir is the "hard shell". This type of venous reservoir has exactly the opposite characteristics of the soft shell variety. The hard shell makes incorporation of an integral cardiotomy reservoir and blood volume measurement easy. It has, however, a free surface of blood exposed to air, and therefore provides no assurance against pumping air when it becomes empty.

It has previously been proposed to facilitate blood measurement in a soft shell reservoir by fabricating the reservoir with one rigid and one flexible side, but that construction does not lend itself to integration of the venous reservoir with a cardiotomy reservoir.

A need therefore exists for a venous reservoir which can be integrated with a cardiotomy reservoir and allows simple, accurate measurement of the blood volume in the reservoir, yet does not expose venous blood to air, and prevents the pumping of air into the extracorporeal blood circuit if the reservoir becomes empty.

The invention fills the above-described need by providing a venous reservoir, comprising:
a) a rigid bowl having an upper end and a lower end;
b) a flexible, blood-impermeable liner sealingly attached to said bowl along the periphery thereof at said upper end, and shaped to lie against substantially the entire inner surface of said bowl when said bowl is empty; and
c) a blood inlet to said bowl, and a blood outlet from said bowl, under said liner at said lower end.

Venous blood and filtered cadiotomy blood are introduced between the hard shell and the liner. The top of the liner is sealed to the hard shell of the venous reservoir. As blood is introduced, the liner rolls up to increase the space between the hard shell and the liner. Thus, the liner cooperates with the hard shell of the venous reservoir to function as a soft shell reservoir within the hard shell reservoir. Blood volume can be easily measured by observing blood level graduations marked on the hard shell. If the reservoir is emptied, the flexible lining collapses against the blood outlet and prevents any air from entering into the outlet.

Preferred embodiments will now be described, by way of example only, with reference to the drawings.
Fig. 1 is a vertical section through an empty venous reservoir constructed in accordance with the invention;
Fig. 2 is a vertical section like Fig. 1 but showing the reservoir partly filled;
Fig. 3 is a vertical section through a combined cardiotomy and venous reservoir in accordance with the invention; and
Fig. 4 is a horizontal section along line 4-4 of Fig. 3.

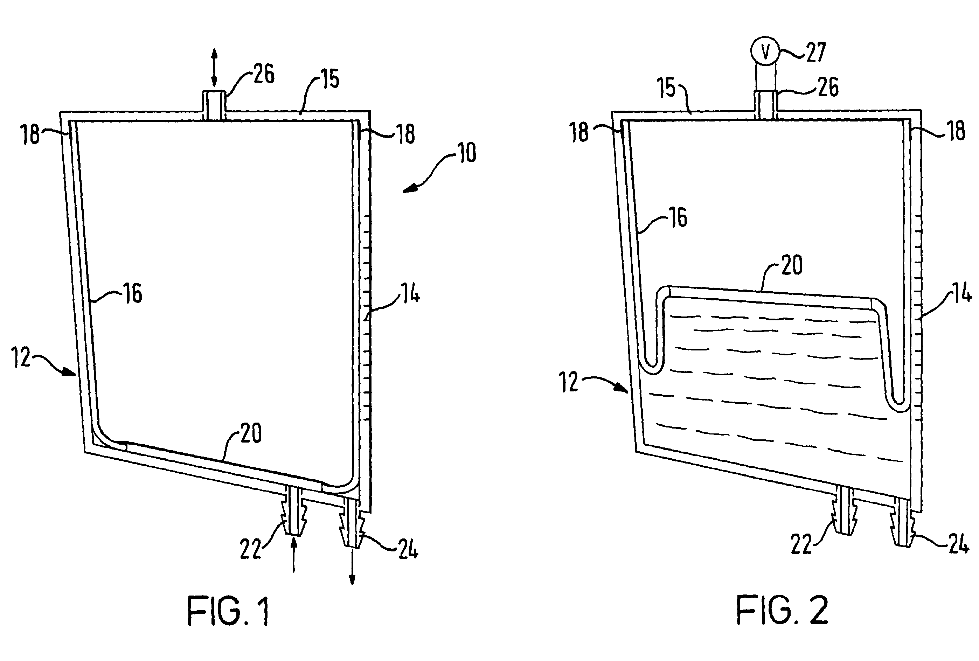

Fig. 1 shows the reservoir 10 of this invention in its empty condition. The reservoir 10 includes a rigid, preferably transparent hard shell bowl 12 equipped with graduations 14 indicating the volume of blood in the reservoir when blood rises to a given level. The bowl 12 is closed off by a cover 15. An inner liner 16 in the form of a sack of flexible, blood-impermeable, preferably transparent plastic material is sealingly secured to the bowl 12 around its circumference at 18, and is so dimensioned that, when the reservoir 10 is empty, it lies snugly against the walls and bottom of the bowl 12.

The central bottom section 20 is formed as a buoyant, rigid plate sealingly attached around its circumference to the liner 16. A blood inlet 22 and a blood outlet 24 are both positioned at the bottom of the bowl 12. By applying a vacuum 27 (Fig. 2) to the purge port 26, assisted venous return can be easily accomplished.

When venous blood is introduced into the bowl 12 through the inlet 22, it pushes the plate 20 upward, and the plate 20 forms the liner 16 into a rolling bellows around its circumference, as best shown in Fig. 2. In the position of Fig. 2, the amount of blood in the reservoir can be readily determined by observing through the transparent bowl 12 and liner 16, the blood level at the plate in relation to the graduations 14. The liner 16 may also be a composite containing a biased fabric to enhance the ease of movement of the rolling bellows.

If blood is pumped out of the outlet 24 more rapidly than blood flows into the reservoir 10 through inlet 22, the plate 20 and liner 16 will eventually return to the position of Fig. 1, in which the liner 16 blocks the outlet 24. Inasmuch as the liner 16 acts as a softshell reservoir which contains only blood, no air can be drawn into the outlet 24 when the reservoir 10 is empty.

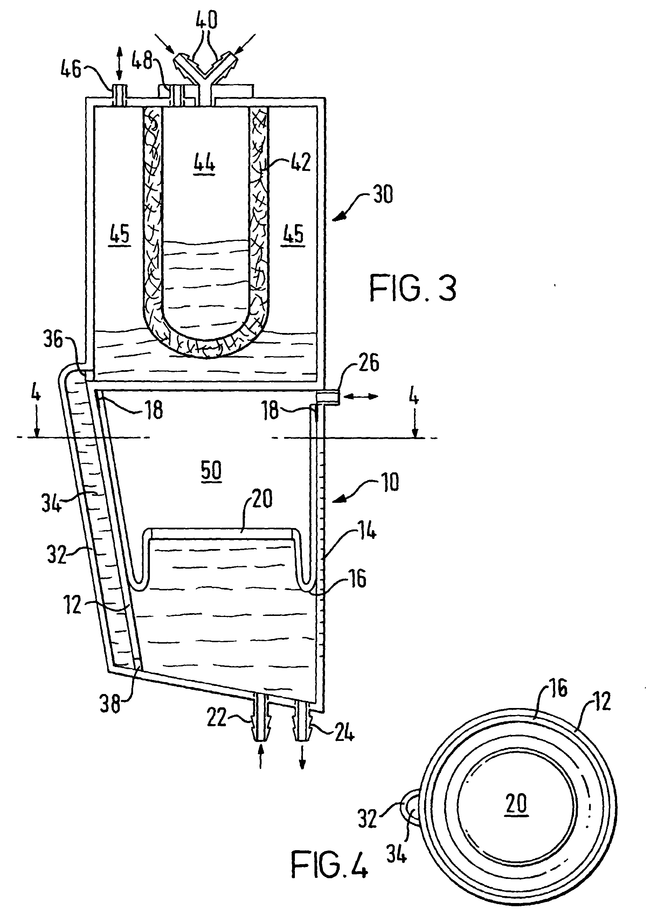

Figs. 3 and 4 show how the inventive venous reservoir 10 can easily be combined with a cardiotomy reservoir. In Fig. 3, the bottom of the cardiotomy reservoir 30 forms the cover for the venous reservoir 10. A hollow rib 32 is formed integrally with the cardiotomy reservoir 30 and the venous reservoir 10 to conduct blood through channel 34 from an outlet 36 at the bottom of the cardiotomy reservoir 30 to an inlet 38 at the bottom of venous reservoir 10.

Cardiotomy blood is conventionally conveyed through the connectors 40 to the interior 44 of filter/defoamer 42. The filtered and defoamed cardiotomy blood collects in the area 45 around and below the filter/defoamer 42. Purge/vacuum ports 46, 48 vent the areas 44 and 45 to atmosphere, while port 26 vents the area 50 of the venous reservoir 10.

## Claims

1. A venous reservoir, comprising:
a) a rigid bowl (12) having an upper end and a lower end;
b) a flexible, blood-impermeable liner (16) sealingly attached to said bowl along the periphery thereof (18) at said upper end, and shaped to lie against substantially the entire inner surface of said bowl when said bowl is empty; and
c) a blood inlet (22) to said bowl, and a blood outlet (24) from said bowl, under said liner at said lower end.

2. The reservoir of Claim 1, further comprising:
d) a substantially rigid plate (20) forming a central portion of said liner (16), the liner surrounding said plate forming a rolling bellows when said plate is pushed upward by the introduction of blood underneath said liner.

3. The reservoir of Claim 1 or 2, in which said bowl (12) and liner (16) are formed of substantially transparent materials.

4. The reservoir of any preceding Claim, further comprising:
e) a cardiotomy reservoir (30) positioned above said bowl (12) and integral therewith; and
f) a conduit (34) for conveying filtered cardiotomy blood from said reservoir to the bottom of said bowl (12) underneath said liner (16).

5. The reservoir of Claim 4, in which said conduit (34) is integral with said cardiotomy reservoir (30) and bowl (12).

6. The reservoir of any preceding claim, wherein said bowl (12) is closed, said reservoir further comprising:
means (27) for applying a vacuum to the upper surface of said liner (16).

7. The reservoir of any of claims 1 to 5, wherein the upper surface of said liner (16) is open to atmosphere.
